# EUROPEAN PATENT SPECIFICATION

(11) **EP 4 327 849 B1**
(45) Date of publication and mention of the grant of the patent: **25.11.2026**
(21) Application number: 23220455.2
(22) Date of filing: 05.03.2013
(51) Int. Cl.: A61M 5/20, A61M 5/00, A61M 5/50, A61M 5/32

(54) **PREFILLED SYRINGE WITH BREAKAWAY FORCE FEATURE**
VORGEFÜLLTE SPRITZE MIT LOSBRECHKRAFTFUNKTION
AMPOULE-SERINGUE À CARACTÉRISTIQUE DE FORCE DE RUPTURE

(30) Priority: 06.03.2012 US 201261607339 P
(43) Date of publication of application: 28.02.2024
(62) Document number(s) of the earlier application(s) in accordance with Art. 76 EPC: 13757994.2 / 2 822 618
(73) Proprietor: Antares Pharma, Inc., Ewing, NJ 08628 (US)
(72) Inventor: LANNAN, James, W., Brooklyn Center, 55429 (US); SUND, Julius, C., Plymouth, 55447 (US)
(74) Representative: Grünecker Patent- und Rechtsanwälte PartG mbB

(56) References cited:
- WO-A1-03/095003
- US-A- 5 681 291
- US-A- 6 102 896
- US-A1- 2007 112 310
- US-A1- 2011 144 594
- US-A1- 2011 208 119
- US-A1- 2012 004 608

## Description

### BACKGROUND OF THE INVENTION

The present invention relates to a jet injector and in some embodiments a needle-assisted jet injector that uses a low jet injection pressure and has a lock ring that provides breakaway force resistance.

Certain jet injection devices have needle guards that must be retracted prior to insertion of the needle and triggering of the jet injection. A certain amount of force is normally required to trigger the jet injection. To assure sufficient needle guard travel for needle insertion and triggering, it is at times desirable to require a breakaway force prior to significant needle guard retraction to assure that insertion of the needle and triggering of triggering force is overcome. The present invention addresses this problem. Further relevant prior art are described in US 2007 112310 A1, US 5 681 291 A, US 2011 208119 A1, US 2011 144594 A1, US 2012 004608 A1, US 6 102 896 A and WO 03 095003 A1.

### SUMMARY OF THE INVENTION

The present invention provides an injector having the features defined in claim 1. Further preferred embodiments are defined in the dependent claims. In certain embodiments, the invention relates to a jet injector. In one embodiment, the jet injector includes a prefilled syringe having a container portion defining a fluid chamber containing a medicament; an injection-assisting needle disposed at the distal end of the chamber, having an injecting tip configured for piercing an insertion location, and defining a fluid pathway in fluid communication with the chamber for injecting the fluid from the chamber into an injection site; a plunger movable within the fluid chamber; a housing that houses the prefilled syringe and is configured for allowing insertion of the needle at the injection location to an insertion point that is at a penetration depth below the surface, the housing including: a retractable guard that is movable between a protecting position in which the needle is disposed within the guard and an injecting position in which the tip of the needle is exposed for insertion to the insertion point, and an interference component adjacent to the retractable guard that interferes with the movement of the retractable guard when the retractable component is moved at least partially from the protecting position toward the injecting position; a syringe support supportively mounting the prefilled syringe in the housing; an energy source configured for biasing the plunger with a force selected to produce an injecting pressure on the medicament in the fluid chamber to jet inject the medicament from the fluid chamber through the needle to the injection site.

In certain embodiments, the energy source and prefilled syringe are configured such that the injecting pressure remains between about 5.52 bar (80 p.s.i.) and about 68.95 bar (1,000 p.s.i.). during injection of the medicament. In one embodiment, the energy source and prefilled syringe are configured such that the injecting pressure remains below about 34.47 bar (500 p.s.i.) and above about 6.21 bar (90 p.s.i.) during the injection of the medicament. In another embodiment, the energy source and prefilled syringe are configured to produce the injecting pressure that remains at least at about 6.89 bar (100 p.s.i.) during the injection of the medicament. In one embodiment, the energy source and prefilled syringe are configured such that the injecting pressure remains up to about 24.13 bar (350 p.s.i.) during the injection of the medicament.

In certain embodiments, the prefilled syringe has a distal portion in which the injection-assisting needle is located, and a proximal portion opposite the distal portion; and the syringe support axially supports the proximal portion of the pre-filled syringe during the jet injection of the medicament, such that the distal portion of the prefilled syringe is substantially unsupported in an axial direction. In one embodiment, the container portion of the pre-filled syringe is made of blown glass. In another embodiment, the injection-assisting needle is adhered to the glass.

In certain embodiment, the interference component is a ring having at least one abutment arm extending distally from a proximal end dimensioned to fit within the housing, the abutment arm having at least one tapered portion. In one embodiment, the at least one abutment arm has an engagement portion axially adjacent to the at least one tapered portion that is configured to cause resistance to the movement of the retractable guard when the retractable guard is moved at least partially from the protecting position toward the injecting position.

In one embodiment, the energy source comprises a spring. In one embodiment, the jet injector further includes a ram that is biased by the spring against the plunger to produce the injecting pressure, wherein the ram comprises a bell portion on which the spring is seated, and the bell portion defines a hollow interior configured for receiving the prefilled syringe when the device is fired, such that the spring surrounds the prefilled syringe.

In some embodiments, the jet injector further includes a trigger mechanism operably associated with the energy source for activating the energy source to jet inject the medicament, wherein the trigger mechanism is configured for activating the energy source after the retractable guard is retracted from the protecting position. In one embodiment, the retractable guard is operably associated with the trigger mechanism to cause the trigger mechanism to activate the energy source when the guard is retracted to the injecting position.

In some embodiments, the interference component is a sleeve having an engagement portion extending outwardly from an outer surface of the sleeve that is configured to cause resistance to the movement of the retractable guard when the retractable guard is moved at least partially from the protecting position toward the injecting position. In other embodiments, the interference component is a latch coupled to the housing that is configured to cause resistance to the movement of the retractable guard when the retractable guard is moved at least partially from the protecting position toward the injecting position.

In certain embodiments, the housing is configured for allowing insertion of the needle to the penetration depth, which is between about 0.5 mm and about 5 mm below the surface at the insertion location.

In certain embodiments, the housing is configured for allowing insertion of the needle to the penetration depth, which is between about 11 mm and about 13 mm below the surface at the insertion location.

In certain embodiments, the chamber contains about between 0.02 mL and about 4 mL of the medicament.

In certain embodiments, the penetration depth and injecting pressure are sufficient to substantially prevent backflow of the injected medicament.

In other embodiments, the jet injector further includes a syringe cushion associated with the syringe support and prefilled syringe to compensate for shape irregularities of the pre-filled syringe.

In certain embodiments, the invention relates to a lock ring for a jet injector. In other embodiments, the lock ring includes at least one abutment arm extending distally from a proximal end of a body dimensioned to fit within in a housing of the jet injector, the abutment arm having at least one tapered portion and at least one engagement portion axially adjacent to the at least one tapered portion, the engagement portion being configured to cause resistance to the movement of a retractable guard of the jet injector; and at least one flap radially adjacent to the at least one abutment arm extending distally from the proximal end of the body.

### BRIEF DESCRIPTION OF THE SEVERAL VIEWS OF THE DRAWINGS

The foregoing summary, as well as the following detailed description of embodiments of the present invention, will be better understood when read in conjunction with the appended drawings of exemplary embodiments. It should be understood, however, that the invention is not limited to the precise arrangements and instrumentalities shown.

In the drawings:
FIG. 1 is a side view of an embodiment of a jet injector constructed according to the present invention, showing the injector prior to injection;
FIG. 2 is a cross-sectional view of the jet injector of FIG. 1 thereof taken along plane II-II;
FIG. 3 is a perspective view of a prefilled syringe for use in the jet injector of FIG. 1;
FIG. 4 is a perspective view of a syringe cushion of the jet injector of FIG. 1;
FIG. 5 is a cross-sectional view of the jet injector of FIG. 1, showing the injector at the start of the jet injection;
FIG. 6 is a graph showing the pressure present in the polluted chamber over time that contains medicament in an embodiment during jet injection;
FIG. 7 is a side view of another embodiment of an injector that is configured for using a narrow diameter prefilled syringe;
FIG. 8 is a cross-sectional view of the jet injector of FIG. 1; taken along plane VIII-VIII;
FIG. 9 is a cross-sectional view of another embodiment of an injector using a needle for intramuscular jet-injection;
FIG. 10A is a side view of an interference component of a jet injector in accordance with an exemplary embodiment;
FIG. 10B is a perspective view of an interference component of a jet injector in accordance with an exemplary embodiment; and
FIG. 11 is a graph showing the breakaway force over time of an jet injector in accordance with an exemplary embodiment.

### DETAILED DESCRIPTION OF THE INVENTION

With reference to the accompanying drawings, various embodiments of the present invention are described more fully below. Some but not all embodiments of the present invention are shown. Indeed, various embodiments of the invention may be embodied in many different forms and should not be construed as limited to the embodiments expressly described. Like numbers refer to like elements throughout. The singular forms "a," "an," and "the" include the singular and plural unless the context clearly dictates otherwise.

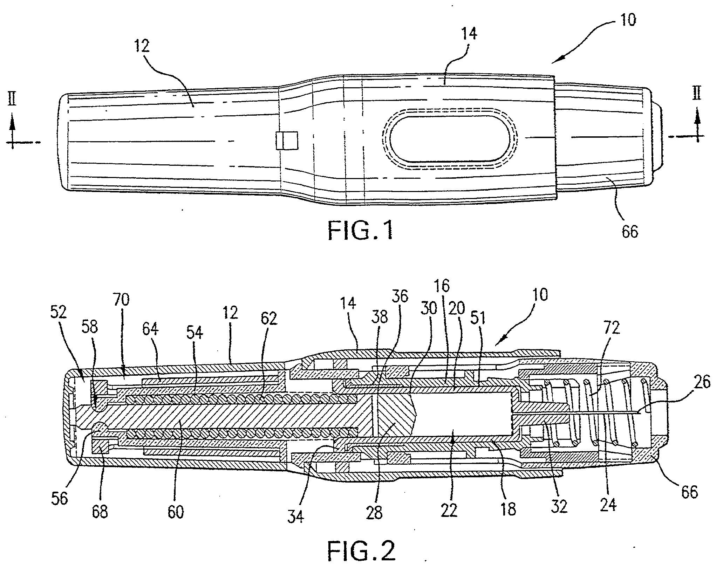

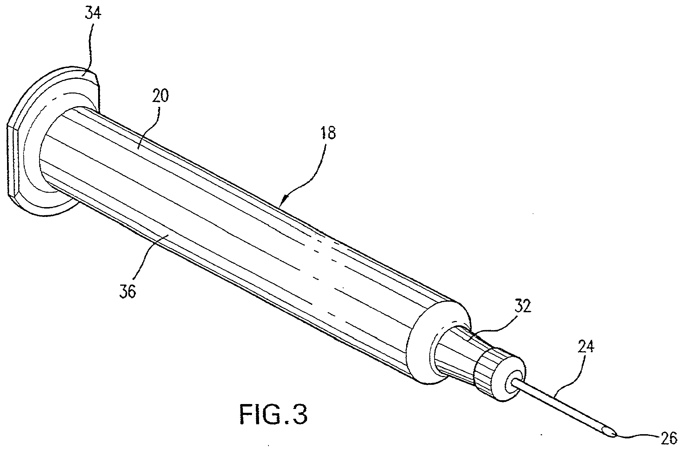

Referring to FIGS. 1 and 2, an embodiment of an injector **10** has a housing **12** configured for allowing a user to handle the injector **10.** The housing **12** includes an outer housing member **14** that substantially houses most of the components shown in FIG. 2. A syringe support member **16** is housed within and mounted with the housing **12.** The syringe support member **16** is configured to hold and position a prefilled syringe **18,** which is shown in FIG. 3. In one embodiment, the syringe support member **16** is substantially fixed to the housing **12,** such as by snaps, an adhesive, a weld, or another known attachment. The prefilled syringe **18** has a container portion **20** that defines in its interior a fluid chamber **22,** which is prefilled with medicament to be injected. At the distal end of the prefilled syringe **18** is an injection-assisting needle **24.** Needle **24** has an injecting tip **26** configured as known in the art to penetrate the tissue of a patient, in certain embodiments, the skin of the patient. A needle bore extends through the needle **24,** as known in the art. The bore is in fluid communication with the medicament in the fluid chamber **22** and is open at the needle tip **26** to inject the medicament.

At a proximal side of the fluid chamber **22,** opposite from the needle **24,** is a plunger **28** that seals the medicament in the fluid chamber **22.** In certain embodiments, a syringe wall **30** comprises a tubular portion, in some embodiments closed at a distal end and open at a proximal end, to define the fluid chamber **22.** Plunger **28** is slideably received in the tubular portion. The prefilled syringe **20** is configured such that when the plunger **28** is displaced in a distal direction, the volume of the fluid chamber **22** is decreased, forcing the medicament out therefrom and through the bore of the needle **24.**

At the distal end of the fluid chamber **22** is a needle hub portion **32** to which the needle is mounted. In one embodiment, a syringe flange **34** extends radially from the proximal end of the syringe wall **30.**

In one embodiment, the syringe **18** has a syringe body **36** that includes the flange **34,** wall **30** and hub portion **32.** In one embodiment, syringe body **36** that includes flange **34,** wall **30,** and hub portion **32** is of unitary construction. A preferred material for the syringe body **36** is glass, but other materials can be used in other embodiments. A suitable prefilled syringe is the BD Hypak^{™}, which is available in various sizes and volumes and is sold prefilled with medicament. The glass of the syringe body **36** is adhered to the needle **24.** Typical medicaments and medicament categories include epinephrine, atropine, sumatriptan, antibiotics, antidepressants, and anticoagulants. Using a prefilled syringe **18** facilitates handling of the medicament when the injector **10** is assembled, and there is an extensive body of knowledge of how the medicaments keep and behave in a prefilled syringe.

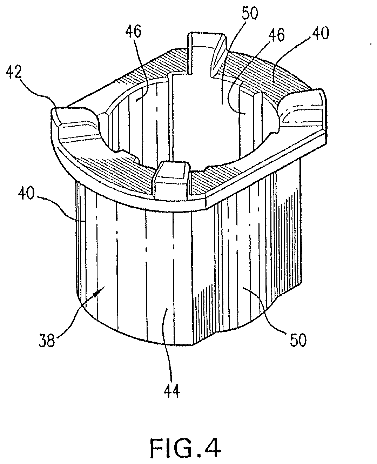

A syringe cushion **38,** which is shown in detail in FIG. 4, is in certain embodiments made of an elastomeric material or other resilient material. A flange **40** of the syringe cushion **38** extends radially and is disposed and serves as an interface between the distal side of the syringe support member **16** and the syringe flange **34.** Elevated portions, such as nubs **42** extend proximately from the cushion flange **40** and are configured and dimensioned to abut the syringe flange **34.**

Prefilled syringes that are manufactured by a blown glass process can have significant dimensional tolerances and unevenness, particularly in the glass body **36.** The cushion **38** can serve to accommodate the shape irregularities and to properly position and locate the prefilled syringe **18** within the syringe support **16.** Typically, the axial thickness of glass blown syringe flanges on a 1 mL prefilled syringe is within about ±0.5 mm. For a BD Hypak^{™} 1 mL standard prefilled syringe, the thickness of the syringe flange **34** is 2 mm+0.5 mm or -0.4 mm, and in a 1 mL long configuration BD Hypak^{™} syringe, the flange axial thickness is about 1.65 mm±0.25 mm. Other dimensional variations that occur in typical glass prefilled syringes are in the internal and external diameters of the tubular wall **30.** These variations can be accommodated by the resilient sleeve portion **44** of the syringe cushion **38,** which extends axially around the interior of the syringe support **16.** In one embodiment, the syringe cushion **38** is received in the interior of the syringe support member **16** and receives the syringe body **36,** in certain embodiments fitting snugly therein.

In one embodiment, the sleeve portion **44** has radially inwardly extending protrusions **46** with a surface area and configuration selected to allow the insertion of the prefilled syringe **18** therein during assembly, but providing sufficient friction to maintain the syringe **18** in place and to provide cushioning and shock absorption during the firing of the injector **10.** Outward protrusions **48** are also provided on the sleeve portion **44,** which can be received in corresponding recesses of the syringe support **16** to prevent axial rotation therebetween. Recessed areas **50** can be provided on the interior and exterior of the syringe cushion **38** opposite corresponding protrusions **48** on the opposite radial side of the sleeve portion **44** if an increased wall thickness of the sleeve portion **44** is not desired. In an alternative embodiment one or both of the flange **40** and sleeve **44** of the syringe cushion **38** are substantially smooth, substantially without any protrusions. In one embodiment, the material and configuration of the syringe cushion **38** is also sufficient to entirely support the prefilled syringe **20** to withstand a firing force applied axially in a distal direction on the plunger **28.** Thus, the entire support for the prefilled **20** can be provided on the syringe flange **34,** while the distal end of the syringe **18** may itself be substantially unsupported in an axial direction. This can help withstand the shock on the glass body **36** of the prefilled syringe **20** produced by the elevated pressures within the fluid chamber **22.**

To radially position the distal end of the prefilled syringe **18,** the syringe support **16** in certain embodiments has a narrowed bore portion **51** that is in certain embodiments configured to abut the outside of the syringe wall **30.** This is especially beneficial when the needle **24** is inserted into the patient's skin. The narrowed bore portion **51** can be made of a resilient material, such as an elastomer, or it can be made unitarily with the rest of the syringe support **16,** in certain embodiments of a plastic material.

Referring to FIG. 2, in one embodiment, a trigger mechanism **52** is also housed within housing **12.** The trigger mechanism **52** includes an inner housing **54** that can be attached to the outer housing **14,** such as by snaps, an adhesive, a weld, or other known attachment. Trigger protrusions **56** extend inwardly from the proximal end of the inner housing **54** and are resiliently biased outwardly. Trigger protrusions **56** are received in a recess **58** of ram **60** in blocking association therewith to prevent distal movement of the ram **60** prior to the firing of the device. The ram **60** is urged towards the distal end of the injector **10** by an energy source, which in certain embodiments is a compression spring **52,** although other suitable energy sources can alternative be used such as elastomer or compressed-gas springs. In one embodiment, the compression spring is a coil spring.

A trigger member of the trigger mechanism **52,** such as a latch housing **64,** is provided exterior to the inner housing to retain the trigger protrusions **56** in the blocking association in the recess **58** to prevent premature firing of the injector **10.** The latch housing **64** is slideable inside the outer housing **14** with respect to the inner housing **54,** in certain embodiments in an axial direction, and the latch housing **64** in certain embodiments surrounds the inner housing **54.**

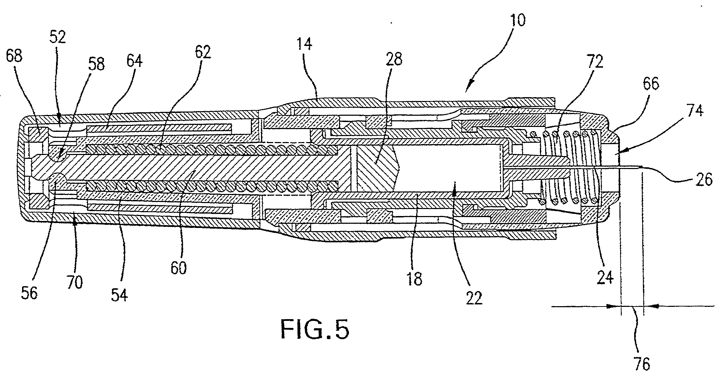

The housing **12** has a needle guard **66** that is moveable with respect to the outer housing **14.** The needle guard **66** is shown in FIGS. 1 and 2 in a protecting position, in which the needle **24** is disposed within the guard **66.** The needle guard **66** is retractable, in one embodiment into the outer housing **14,** in a proximal direction to an injecting position, in which the needle tip **26** and an end portion of the needle **24** is exposed as shown in FIG. 5 for insertion into a patient. In one embodiment, the proximal movement of the guard is prevented substantially at the injecting position.

In one embodiment, an interference component **134** interferes with the movement of the needle guard when the needle guard is moved at least partially from the protecting position toward the injecting position.

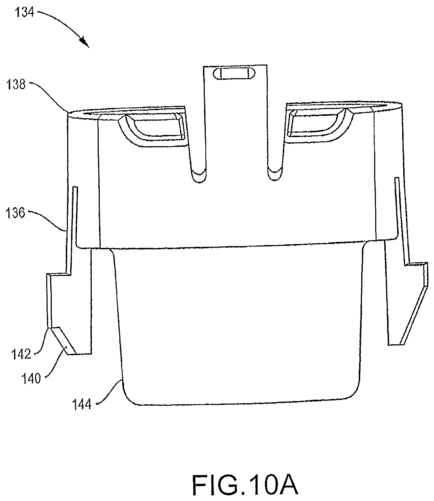

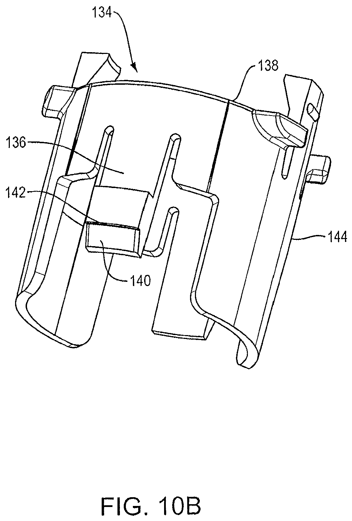

In one embodiment, the housing **12** has an interference component **134,** e.g., a lock ring, adjacent to the needle guard **66,** the interference component **134** interferes with the movement of the needle guard when the needle guard is moved at least partially from the protecting position toward the injecting position. Interference component prevents movement of the needle guard until the breakaway force **146** is exceeded. The interference component **134** is shown in FIGS. 10A and 10B. In one embodiment, the interference component **134** is included as part of a ring having at least one abutment arm **136** extending distally from a proximal end **138** dimensioned to fit within the housing **14,** the abutment arm **136** having at least one tapered portion **140.** The abutment arm **136** may also have an engagement portion **142** axially adjacent to the at least one tapered portion **140** that is configured to cause resistance to the movement of the needle guard **66** when the needle guard **66** is moved at least partially from the protecting position toward the injecting position. While interference component **134** may have more than one abutment arm **136** and correspondingly more than one engagement portion **142,** certain embodiments include only one abutment arm **136** having an engagement portion **142.** The interference component may also include at least one flap **144** radially adjacent to the at least one abutment arm **136** extending distally from the proximal end **138** of the interference component **134.**

The interference component **134** may also be coupled to the housing **12,** incorporated in a sleeve separate from the housing **12,** or include a latch.

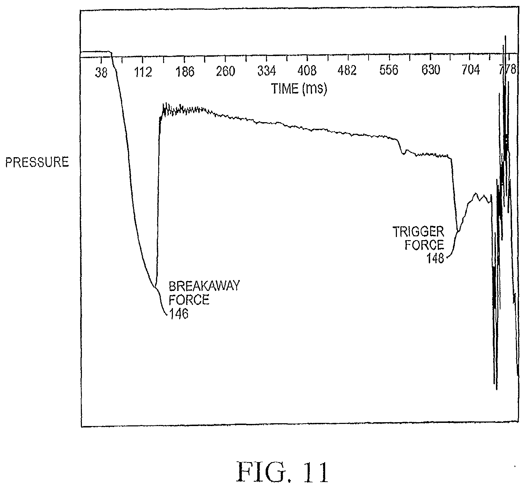

Referring to FIG. 11, breakaway force **146** is needed to overcome the resistance on the needle guard **66** caused by the engagement portion **142** when the needle guard **66** is moved at least partially from the protecting position toward the injecting position. Referring to Fig. 11, breakaway force **146** is the resistance to retraction that is exerted on the needle guard **66** when an initial attempt to retract the needle guard **66** occurs. Breakaway force **146** is a distinct force from the triggering force **148** that is needed to cause jet injection of the medicament and is a greater force than that provided by the spring **62** that biases the needle guard **66** in the extended position. Breakaway force **146** is sometimes also a greater force than what occurs due to the friction of the needle guard **66** retracting motion sliding on other mating components in the device. In one embodiment the breakaway force **146** is controlled and only occurs as a single event.

Referring to FIG. 2, the needle guard **66** is associated with the latch housing **64** such that when the guard **66** is displaced distally it slides the latch housing **64** also in a distal direction to release the trigger protrusions **56** from the recess **58.** In one embodiment, the latch housing **64** has a latching portion **68** that abuts the inner housing **54** in an association to bias and maintain the trigger protrusions **58** positioned in the blocking association with the ram **60** prior to the firing of the device **10.** When the latch is slid proximately by the retracting of the guard **66** to the injecting position, the latching portion **68** slides beyond the portion of inner housing **54** that is contacts to flex the trigger protrusions **56** into the recess **58** of the ram **60,** allowing the trigger protrusions **56** to move radially outwardly from the recess **58** and therefore from the blocking association. When this happens, spring **62** biases the ram **60** against plunger **28** to fire the jet injector **10.** In certain embodiments, latch housing **64** defines trigger openings **70** adjacent to latching portions **68,** which is configured to receive a portion of the inner housing **54,** such as the surface disposed radially outwardly from the trigger protrusions **56.**

In certain embodiments, the guard **66** is resiliently biased distally towards the protecting position by compression coil spring **72.** Also, the needle guard **66** has an axial opening **74** to allow the needle **24** pass there through, and which may be sized according to the type of injector desired. The construction of the present embodiment allows a user to push the distal end of the injector **10** against the patient's skin, pushing the needle **24** into the skin at an insertion location, substantially at the same speed as the injector is pushed. Once the needle **24** is fully inserted to an insertion point at a penetration depth, the trigger mechanism **56** fires the jet injection to an injection site.

Referring to FIG. 5, in one embodiment, the prefilled syringe **18** and its needle **24** are not shuttled forward automatically into the patient's skin, such as by the firing energy source during the injection firing. The user preferably gently pushes the entire device forward to insert the needle **24,** in certain embodiments retracting a guard against the skin in the process. In one embodiment, the prefilled syringe **18** is substantially stationary within the housing **12,** and, in one embodiment, is substantially fixed thereto. In this manner, the present invention provides for a gentler treatment of the syringe during injection that enables the use of a sufficiently powerful spring **62** or other energy source to produce a jet injection without the risk of damaging the relatively fragile and complex shapes of the prefilled syringe, also allowing, for example, the injection of high viscosity solutions, where the risk of breaking a syringe, such as at the flange, is elevated in prior art injectors that shuttle the syringe forward in the housing and into the patient. Residual stresses are also often present in the glass bodies of prefilled syringes, and this configuration reduces the additional stresses imposed thereon during use, further protecting the syringe. Also, misalignments in the prefilled syringe are also rendered operationally less significant due to the gentle insertion of the needle that is possible with this configuration.

In one embodiment, the injecting position of the guard **66** is such that a predetermined length of the end of needle **24** is exposed from the guard **66.** In some embodiments, such as where the opening **74** is of a sufficiently large diameter, the skin of the patient maybe allowed to extend into the opening **74** when the device **10** is pressed there against, and a needle that does not protrude beyond the distal end of the guard **66** can be used while still penetrating the skin to a certain depth. In most embodiments, the distance **76** by which the needle tip **26** extends past the distal end of the guard **66** will be fairly close to the depth of the insertion of the needle.

In one embodiment, such as for subcutaneous injection, the guard **66** is configured to allow insertion of the needle **24** to a penetration depth in the skin that is up to about 5 mm below the skin surface. In another embodiment, the penetration depth is less than about 4 mm, and in one embodiment is less than about 3 mm. In one embodiment, the insertion depth is at least about 0.5 mm and, in other embodiments, at least about 1 mm. In another embodiment, the distance **76** by which the needle extends past the guard **66** or the distal surface of the guard **66** that contacts the skin is up to about 5 mm, in one embodiment, up to about 4 mm, and in another embodiment up to about 3 mm. In certain embodiments, extension distance **76** is at least about 0.5 mm, in one embodiment at least about 1 mm, and in another embodiment at least about 2 mm. In one embodiment, tip **26** extends by a distance **76** of around 2.5 mm beyond the portion of the guard **66** that contacts the skin in the injecting position.

In another embodiment, such as for intramuscular injection, the injector is configured to allow the needle **24** to be inserted into the patient to a penetration depth in the skin, or alternatively beyond the distal surface of the guard, by a distance of up to about 15 mm. In one embodiment, this distance is about between 10 mm and 14 mm. In an embodiment for jet injection of epinephrine for instance, a penetration depth or distance beyond the guard is between about 11 mm and about 17.0 mm, and, in other embodiments, between about 13 to about 15 mm. Jet injection with this length needle improves the distribution of the medicament in the patient tissue compared to non-jet injection. Other exposed needle lengths can be selected for jet injection to different depths below the skin, with, in certain embodiments, an overall penetration length of between about 0.5 mm and about 20 mm. In certain embodiments, the needle guard is configured for retracting from a protecting position, in one embodiment covering the entire needle **24** (See FIG. 2), to an injecting position, in which the desired length of the end of the needle **24** is exposed (See FIG. 5).

In some embodiments, the spring **62** and the prefilled syringe **18** are configured to jet inject the medicament. Thus, the spring **62** applies a force on the plunger **28** that is sufficient to elevate the pressure within the fluid chamber **22** to a level high enough to eject the medicament from the needle **24** as a jet. Jet injection is to be understood as an injection with sufficient velocity and force to drive the medicament to locations remote from the needle tip **26.** In manual and autoinjector-type injections, in which the injection pressures are very low, the medicament exits the needle tip inside the patient and is typically deposited locally around the needle in a bolus. On the other hand, with the present jet injection device **10,** the medicament is jet injected distally or in other directions, such as generally radially by the elevated pressure jet, which beneficially improves the distribution of the medicament after the injection and keeps a large bolus from forming that can detrimentally force the medicament to leak back out of the patient around the needle or through the hole left behind by the needle after it is removed.

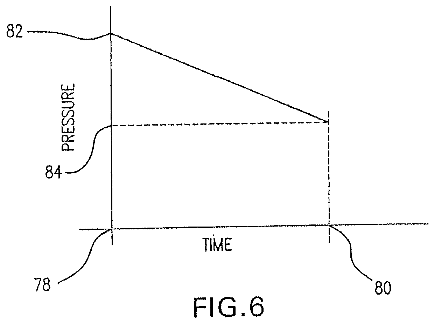

Referring to the graph shown in FIG. 6, numeral **78** represents the point in time when device **10** is fired, and numeral **80** represents the point in time of completion of the medicament injection, in certain embodiments when the plunger **28** hits the forward wall of the container portion **20.** Numeral **82** represents the initial and peak pressure during the injection, and numeral **84** represents the final and low pressure during the injection. Since the spring **62** of one embodiment has a linear spring constant and an injection-assisting needle is used to puncture the skin before commencing the injection, the pressure drops substantially linearly from the start of the injection **78** until the injection is completed. The final pressure **84** at the end **80** of the injection is sufficiently elevated so that even at the end of the firing stroke of ram **60,** the medicament is still jet injected, and a very small amount or none of the medicament is deposited in a bolus around the needle tip **26.**

In one embodiment, the peak pressure during the injection is less than about 68.95 bar (1000 p.s.i), in one embodiment less than about 34.47 bar (500 p.s.i.), and in another embodiment less than about 24.13 bar (350 p.s.i.). At the end **80** of the injection, the pressure **84** applied to the medicament in the fluid chamber **22** is in one embodiment at least about 5.52 bar (80 p.s.i.) in one embodiment at least about 6.21 bar (90 p.s.i.), and in another embodiment at least about 6.89 bar (100 p.s.i.). In one embodiment of the invention, the initial pressure **82** is around 22.75 bar (330 p.s.i.) and the final pressure is about 12.41 bar (180 p.s.i.), while in another embodiment the initial pressure **82** is about 20.68 bar (300 p.s.i.), dropping to around 7.58 bar (110 p.s.i.) at the end **80** of the injection. The needles used in these embodiments are between 26 and 28 gauge, and are in certain embodiments around 27 gauge, but alternatively other needle gages can be used where the other components are cooperatively configured to produce the desired injection. In an embodiment for jet injection of epinephrine for instance, certain embodiments of the needles are between 20 and 25 gauge, and in other embodiments, 22 gauge. In one embodiment, the components of the injector **10** are configured to jet inject the medicament to a subterraneous injection site.

The amount of medicament contained and injected from fluid chamber **22** is in one embodiment between about 0.02 mL and about 4 mL, in certain embodiments less than about 3 mL, and in other embodiments is around 1 mL. Larger volumes may also be selected depending on the particular medicament and dosage required. In one embodiment, the prefilled syringe is assembled into the remaining parts of the jet injector **10** already containing the desired amount of medicament. In one embodiment, the prefilled syringe contains about 1 mL of medicament.

In one embodiment, injection rates are below about 0.75 mL/sec., in one embodiment preferably below about 0.6 mL/sec., in one embodiment at least about 0.2 mL/sec., in one embodiment at least about 0.3 mL/sec, and in other embodiments at least about 0.4 mL/sec. In one embodiment, the injection of the entire amount of medicament is completed in less than about 4 seconds, in one embodiment in less than about 3 seconds, and in other embodiments in less than about 2.5 seconds. In one embodiment, the medicament injection takes at least about 1 second, in one embodiment at least 1.5 seconds, and in other embodiments at least about 1.75 seconds. In one embodiment, the injector **10** injects the medicament at about 0.5 mL/sec., completing the injection of 1 mL in about 2 seconds.

U.S. Pat. No. 6,391,003 discloses several experimental results of pressures that can be applied to medicament in a glass cartridge, using 26 and 27 gauge needles. The following table illustrates injections with different peak pressures that can be used with glass prefilled syringes:

### Pressure and Time (sec.) to Inject 1 cc

| Pressure | 26 Gauge needle | 27 Gauge needle |
|---|---|---|
| 150 p.s.i. | 2.1 | 4.2 |
| 200 p.s.i. | 1.9 | 3.9 |
| 240 p.s.i. | 1.7 | 3.3 |
| 375 p.s.i. | 1.4 | 3.1 |

It is foreseen that higher pressures and flow rates will be used with shorter needle penetration into the patient skin to achieve jet injections to a particular desired depth substantially without medicament leakback.

It has been found that using the jet injection of the present device, short needles can be used to inject medicament to different parts of the skin, in certain embodiments subcutaneously, substantially without any leakback. Using a needle **24** that extends by about 2.5 mm from the needle guard **66,** a 27 gauge needle **24,** and a pressure in the fluid chamber **22** peaking at around 20.68 bar (300 p.s.i.) and ending at around 6.89 bar (100 p.s.i.) resulting in a flow rate of about 0.5 mL/sec., 1 mL of medicament has been found to successfully be injected without leakback in close to 100% of the tested injections. Thus, the needle-assisted jet injector **10** of the present invention permits jet injection of the medicament using a very short needle reliably regardless of the thickness of the patient's skin or the patient's age, weight or other typical factors that complicate non-jet injecting with short needles.

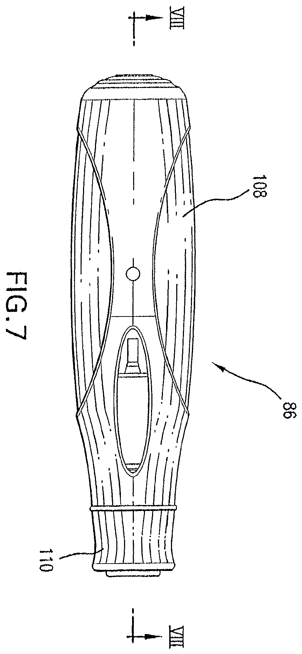

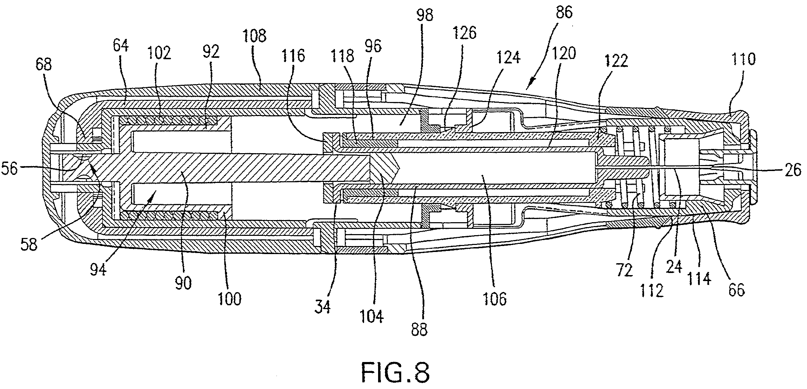

FIGS. 7 and 8 show another embodiment of the present invention that uses a prefilled syringe that has a long, but smaller-diameter configuration than the embodiment of FIG. 2. While in the embodiment of FIG. 2, the firing spring **62** extends into the bore of the prefilled syringe **18** during the firing stroke, the narrower prefilled syringe **88** of injector **86** does not provide as much space to accommodate a spring. Consequently, the ram **90** of injector **86** includes a bell portion **92** defining a hollow interior **94** that is configured to receive the proximal end of the prefilled syringe **88** and the syringe support **96** when the injector **86** is fired. Similarly, a bell-receiving space **98** is defined around the exterior of the prefilled syringe **88** and syringe support **96** to receive the bell portion **92** during the firing. The bell portion **92** includes a spring seat **100** extending radially outwardly and configured and disposed to seat a compression spring **102.** When the trigger mechanism **56** is activated and the device **86** is fired, spring **102** acts against seat **100** to drive the ram **90** against plunger **104** to jet inject the medicament from the fluid chamber **106.** As a result, after firing, the spring **102** radially surrounds the prefilled syringe **88.** The outer housing portion **108** is wider than outer housing portion **14** of injector **10** to accommodate the bell portion **92** and larger diameter spring **102.**

One available long configuration syringe with a 1 mL capacity has a cylindrical syringe body portion with a diameter of 8.15 mm, which would in certain embodiments be used in the injector of FIGS. 7 and 8, while one available shorter configuration syringe of the same capacity has a cylindrical syringe body portion with a diameter of 10.85 mm, which would in certain embodiments be used in the injector of FIGS. 1 and 2. While the embodiment with a bell portion **92** can be used with wider/shorter syringes, in certain embodiments, the prefilled syringes have an outer diameter cylindrical wall of less than about 10 mm, and in other embodiments less than about 9 mm.

Injector **86** also includes a cap **110** fitted around the needle guard **66,** and associated with the outer housing **108** to prevent retraction of the needle guard **66** and the triggering of the device **86.** Additionally, the cap **110** seals off the needle tip **26** and can be removed prior to using the device **86.** In one embodiment, the cap **110** is configured to fit over the needle guard **66** in a snap-fit association therewith, such as by including a narrower diameter portion **112** associated with an enlarged diameter portion **114** of the needle guard **66.**

Additionally, injector **86** employs a syringe cushion cap **116** that extends around the outside of the syringe flange **34** from the syringe cushion **118** to help trap and retain the prefilled syringe **88.** In one embodiment, a cushion cap **122** is connected to the cushion **118** and is, in certain embodiments, of unitary construction therewith. The cushion cap **122** abuts the distal end of the syringe body **120** to radially position and hold the proximal end of the body **120** while the needle **24** is being inserted into the patient. Similarly to the embodiment of FIG. 2, the syringe holder **96** is associated with the housing in a substantially fixed position, such as by mounting portion **124,** which traps protrusions **126** of the syringe holder.

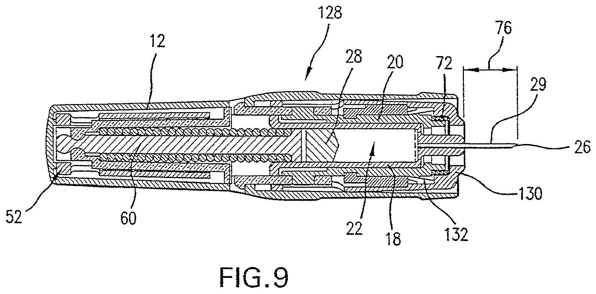

Referring to FIG. 9, injector **128** has a needle guard **130** configured to retract further into the injector housing than the injector of FIGS. 1 and 2 or FIG. 5 before the trigger mechanism **52** fires the jet injection. The injector in this figure is shown in a position in which the trigger mechanism **52** is being released and about to fire the injection. The distance **76** by which the needle extends past the guard **130** or the distal surface of the guard **130** that contacts the skin in certain embodiments between about 12.5 and 13 mm. In one embodiment, the guard is preferably configured to reextend to a protecting position after the device is fired and removed from the patient, such as under the bias of spring **72,** and is locked in that position by locking members **132,** as known in the art to prevent reuse on the injector.

In other embodiments, the guard length, the location of the guard injecting position with respect to the needle tip (including the guard throw between the protecting and injecting positions), and the length of the needle from the syringe body can be selected to allow for shallower or deeper needle insertions before the device is fired, providing lesser or greater distances **76,** respectively. In one embodiment, the guard is kept from sliding further back than substantially at the firing position, to better control in insertion depth into the patient.

## Claims

1. An injector (10), comprising:
a prefilled syringe (18) comprising:
a container portion defining a fluid chamber (22) containing a medicament;
a needle (24) disposed at the distal end of the chamber having an injecting tip configured to deliver the medicament from the fluid chamber (22) into an injection site;
a plunger (28) movable within the fluid chamber (22);
a housing (12) that houses the prefilled syringe (18) and is configured to allow insertion of the needle (24) at an injection location, the housing (12) comprising:
a retractable guard (66) that is movable between a protecting position in which the needle (24) is disposed within the retractable guard (66) and an injecting position in which a tip (26) of the needle (24) is exposed for insertion into the injection site;
a coil spring (72) configured to bias the retractable guard (66) toward the protecting position ([0048]), and
an interference component (134) having at least one abutment arm (136), the abutment arm (136) having at least one tapered portion (140) and an engagement portion (142) axially adjacent to the at least one tapered portion (140), the engagement portion (142) configured to resist the movement of the retractable guard (66) when the retractable guard (66) is moved from the protecting position toward the injecting position; and
an energy source (62) configured to bias the plunger (28) with a force selected to produce an injecting pressure on the medicament in the fluid chamber (22) to inject the medicament from the fluid chamber (22) through the needle to the injection site,
wherein a breakaway force is configured to be applied to the retractable guard (66) to overcome the resistance to movement of the retractable guard (66) caused by the engagement portion (142) of the abutment arm (136),
wherein the breakaway force is distinct from a trigger force, wherein the trigger force causes injection of the medicament.

2. The injector (10) of claim 1, wherein the trigger force is applied to the injection device (10) to cause injection of the fluid from the fluid chamber into the injection site.

3. The injector (10) of claim 1, wherein the breakaway force is greater than a frictional force from friction between the retractable guard (66) and the injection device (10) as the retractable guard (66) moves from the protecting position toward the injecting position.

4. The injector (10) of claim 1, wherein the breakaway force is applied during an initial movement of the retractable guard toward the injecting position.

5. The injector (10) of claim 1, wherein the interference component (136) is directly coupled to the housing of the injection device.

6. The injector (10) of claim 1, wherein the interference component (136) is constituted bya sleeve separate from the housing of the injection device.

7. The injector (10) of claim 1, wherein the interference component (136) is constituted by a lock ring.

8. The injector (10) of claim 1, wherein the injection device is a jet injection device.

9. The injector (10) of claim 1, wherein the engagement portion (142) extends radially outwardly relative to a longitudinal axis of the injection device.

10. The injector (10) of claim 1, further comprising:
a second abutment arm (136) including a second abutment arm tapered portion and a second abutment arm engagement portion axially adjacent to the second abutment arm tapered portion .

## Patentansprüche

1. Injektor (10), umfassend:
eine vorgefüllte Spritze (18), umfassend:
einen Behälterabschnitt, der eine Fluidkammer (22) definiert, die ein Medikament enthält;
eine Nadel (24), die am distalen Ende der Kammer angeordnet ist und eine Injektionsspitze aufweist, die dazu konfiguriert ist, das Medikament aus der Fluidkammer (22) in eine Injektionsstelle abzugeben;
einen Kolben (28), der innerhalb der Fluidkammer (22) beweglich ist;
ein Gehäuse (12), das die vorgefüllte Spritze (18) aufnimmt und dazu konfiguriert ist, das Einführen der Nadel (24) an einer Injektionsstelle zu ermöglichen, wobei das Gehäuse (12) umfasst:
eine einziehbare Schutzvorrichtung (66), die zwischen einer Schutzposition, in der die Nadel (24) innerhalb der einziehbaren Schutzvorrichtung (66) angeordnet ist, und einer Injektionsposition, in der eine Spitze (26) der Nadel (24) zum Einführen in die Injektionsstelle freiliegt, beweglich ist;
eine Schraubenfeder (72), die dazu konfiguriert ist, die einziehbare Schutzvorrichtung (66) in Richtung der Schutzposition vorzuspannen [0048], und
eine Interferenzkomponente (134) mit mindestens einem Anschlagarm (136), wobei der Anschlagarm (136) mindestens einen sich verjüngenden Abschnitt (140) und einen Eingriffsabschnitt (142) aufweist, der axial an den mindestens einen sich verjüngenden Abschnitt (140) angrenzt, wobei der Eingriffsabschnitt (142) dazu konfiguriert ist, der Bewegung der einziehbaren Schutzvorrichtung (66) Widerstand entgegenzusetzen, wenn die einziehbare Schutzvorrichtung (66) von der Schutzposition in Richtung der Injektionsposition bewegt wird; und
eine Energiequelle (62), die dazu konfiguriert ist, den Kolben (28) mit einer Kraft vorzuspannen, die so gewählt ist, dass sie einen Injektionsdruck auf das Medikament in der Fluidkammer (22) erzeugt, um das Medikament aus der Fluidkammer (22) durch die Nadel zur Injektionsstelle zu injizieren,
wobei eine Abreißkraft dazu konfiguriert ist, auf die einziehbare Schutzvorrichtung (66) ausgeübt zu werden, um den durch den Eingriffsabschnitt (142) des Anschlagarms (136) verursachten Widerstand gegen die Bewegung der einziehbaren Schutzvorrichtung (66) zu überwinden,
wobei die Abreißkraft von einer Auslösekraft verschieden ist, wobei die Auslösekraft die Injektion des Medikaments bewirkt.

2. Injektor (10) nach Anspruch 1, wobei die Auslösekraft auf die Injektionsvorrichtung (10) ausgeübt wird, um die Injektion des Fluids aus der Fluidkammer in die Injektionsstelle zu bewirken.

3. Injektor (10) nach Anspruch 1, wobei die Abreißkraft größer ist als eine Reibungskraft aufgrund der Reibung zwischen der einziehbaren Schutzvorrichtung (66) und der Injektionsvorrichtung (10), wenn sich die einziehbare Schutzvorrichtung (66) von der Schutzposition in Richtung der Injektionsposition bewegt.

4. Injektor (10) nach Anspruch 1, wobei die Abreißkraft während einer anfänglichen Bewegung der einziehbaren Schutzvorrichtung in Richtung der Injektionsposition ausgeübt wird.

5. Injektor (10) nach Anspruch 1, wobei die Interferenzkomponente (136) direkt mit dem Gehäuse der Injektionsvorrichtung gekoppelt ist.

6. Injektor (10) nach Anspruch 1, wobei die Interferenzkomponente (136) durch eine vom Gehäuse der Injektionsvorrichtung separate Hülse gebildet ist.

7. Injektor (10) nach Anspruch 1, wobei die Interferenzkomponente (136) durch einen Sperrring gebildet ist.

8. Injektor (10) nach Anspruch 1, wobei die Injektionsvorrichtung eine Strahlinjektionsvorrichtung ist.

9. Injektor (10) nach Anspruch 1, wobei sich der Eingriffsabschnitt (142) in Bezug auf eine Längsachse der Injektionsvorrichtung radial nach außen erstreckt.

10. Injektor (10) nach Anspruch 1, ferner umfassend:
einen zweiten Anschlagarm (136), der einen sich verjüngenden Abschnitt des zweiten Anschlagarms und einen Eingriffsabschnitt des zweiten Anschlagarms umfasst, der axial an den sich verjüngenden Abschnitt des zweiten Anschlagarms angrenzt.

## Revendications

1. Injecteur (10), comprenant :
une seringue préremplie (18), comprenant :
une partie conteneur définissant une chambre de fluide (22) contenant un médicament ;
une aiguille (24) disposée à l'extrémité distale de la chambre et ayant une pointe d'injection conçue pour délivrer le médicament depuis la chambre de fluide (22) dans un site d'injection ;
un piston (28) mobile à l'intérieur de la chambre de fluide (22) ;
un logement (12) qui loge la seringue préremplie (18) et qui est conçu pour permettre l'insertion de l'aiguille (24) au niveau d'une localisation d'injection, le logement (12) comprenant :
une protection (66) rétractable qui est mobile entre une position de protection dans laquelle l'aiguille (24) est disposée à l'intérieur de la protection (66) rétractable et une position d'injection dans laquelle une pointe (26) de l'aiguille (24) est exposée pour l'insertion dans le site d'injection ;
un ressort hélicoïdal (72) conçu pour solliciter la protection (66) rétractable vers la position de protection [0048], et
un composant d'interférence (134) ayant au moins un bras de butée (136), le bras de butée (136) ayant au moins une partie effilée (140) et une partie d'engagement (142) axialement adjacente à la au moins une partie effilée (140), la partie d'engagement (142) étant conçue pour opposer une résistance au mouvement de la protection (66) rétractable lorsque la protection (66) rétractable est déplacée depuis la position de protection vers la position d'injection ; et
une source d'énergie (62) conçue pour solliciter le piston (28) avec une force sélectionnée pour produire une pression d'injection sur le médicament dans la chambre de fluide (22) afin d'injecter le médicament depuis la chambre de fluide (22) à travers l'aiguille vers le site d'injection,
une force de séparation étant conçue pour être appliquée à la protection (66) rétractable afin de surmonter la résistance au mouvement de la protection (66) rétractable causée par la partie d'engagement (142) du bras de butée (136),
la force de séparation étant distincte d'une force de déclenchement, la force de déclenchement provoquant l'injection du médicament.

2. Injecteur (10) selon la revendication 1, la force de déclenchement étant appliquée au dispositif d'injection (10) pour provoquer l'injection du fluide depuis la chambre de fluide dans le site d'injection.

3. Injecteur (10) selon la revendication 1, la force de séparation étant supérieure à une force de frottement résultant du frottement entre la protection (66) rétractable et le dispositif d'injection (10) lorsque la protection (66) rétractable se déplace depuis la position de protection vers la position d'injection.

4. Injecteur (10) selon la revendication 1, la force de séparation étant appliquée pendant un mouvement initial de la protection rétractable vers la position d'injection.

5. Injecteur (10) selon la revendication 1, le composant d'interférence (136) étant directement accouplé au logement du dispositif d'injection.

6. Injecteur (10) selon la revendication 1, le composant d'interférence (136) étant constitué par un manchon séparé du logement du dispositif d'injection.

7. Injecteur (10) selon la revendication 1, le composant d'interférence (136) étant constitué par une bague de verrouillage.

8. Injecteur (10) selon la revendication 1, le dispositif d'injection étant un dispositif d'injection par jet.

9. Injecteur (10) selon la revendication 1, la partie d'engagement (142) s'étendant radialement vers l'extérieur par rapport à un axe longitudinal du dispositif d'injection.

10. Injecteur (10) selon la revendication 1, comprenant en outre :
un second bras de butée (136) comprenant une partie effilée du second bras de butée et une partie d'engagement du second bras de butée axialement adjacente à la partie effilée du second bras de butée.
